(19) Europäisches Patentamt
European Patent Office
Office européen des brevets

(11) EP 1 487 453 B1

(12) # EUROPEAN PATENT SPECIFICATION

(45) Date of publication and mention of the grant of the patent:
**13.01.2010 Bulletin 2010/02**

(21) Application number: **03712117.5**

(22) Date of filing: **27.03.2003**

(51) Int Cl.:
***A61K 31/5375*** *(2006.01)* ***C07D 265/30*** *(2006.01)*

(86) International application number:
**PCT/EP2003/003339**

(87) International publication number:
**WO 2003/082291 (09.10.2003 Gazette 2003/41)**

(54) **N- ¬(2S)-4-(3,4-DIFLUOROBENZYL)MORPHOLIN-2-YL METHYL -2- 3-¬(METHYLSULPHONYL) AMINO PHENYL ACETAMIDE AS CCR3 ANTAGONIST FOR THE TREATMENT OF INFLAMMATORY CONDITIONS**

N- ¬(2S)-4-(3,4-DIFLUOROBENZYL)MORPHOLIN-2-YL METHYL -2- 3-¬(METHYLSULPHONYL) AMINO PHENYL ACETAMID ALS CCR3 ANTAGONIST FÜR DIE BEHANDLUNG VON ENTZÜNDLICHEN ERKRANKUNGEN

N- (2S) -4- (3, 4-DIFLUOROBENZYLE) MORPHOLINE-2-YLE]METHYLE -2- 3-(METHYLSULPHONYLE) AMINO] PHENYLE ACETAMIDE UTILISE EN TANT QU'ANTAGONISTE DU RECEPTEUR CCR3 POUR LE TRAITEMENT D'ETATS INFLAMMATOIRES

(84) Designated Contracting States:
**AT BE BG CH CY CZ DE DK EE ES FI FR GB GR HU IE IT LI LU MC NL PT RO SE SI SK TR**
Designated Extension States:
**LT LV**

(30) Priority: **28.03.2002 GB 0207449**

(43) Date of publication of application:
**22.12.2004 Bulletin 2004/52**

(73) Proprietor: **GLAXO GROUP LIMITED**
**Greenford, Middlesex UB6 ONN (GB)**

(72) Inventors:
- **ANCLIFF, Rachael, Ann, GlaxoSmithKline Stevenage, Hertfordshire SG1 2NY (GB)**
- **COOK, Caroline, Mary, GlaxoSmithKline Stevenage, Hertfordshire SG1 2NY (GB)**
- **ELDRED, Colin, David, GlaxoSmithKline Stevenage, Hertfordshire SG1 2NY (GB)**
- **GORE, Paul, Martin, GlaxoSmithKline Stevenage, Hertfordshire SG1 2NY (GB)**
- **HARRISON, Lee, Andrew, GlaxoSmithKline Stevenage, Hertfordshire SG1 2NY (GB)**
- **HAYES, Martin, Alistair, GlaxoSmithKline Stevenage, Hertfordshire SG1 2NY (GB)**
- **HODGSON, Simon, Teanby, GlaxoSmithKline Stevenage, Hertfordshire SG1 2NY (GB)**
- **JUDD, Duncan, Bruce, GlaxoSmithKline Stevenage, Hertfordshire SG1 2NY (GB)**
- **KEELING, Suzanne, Elaine, GlaxoSmithKline Stevenage, Hertfordshire SG1 2NY (GB)**
- **LEWELL, Xiao, Qing, GlaxoSmithKline Stevenage, Hertfordshire SG1 2NY (GB)**
- **MILLS, Gail, GlaxoSmithKline Stevenage, Hertfordshire SG1 2NY (GB)**
- **ROBERTSON, Graeme, Michael, GlaxoSmithKline Stevenage, Hertfordshire SG1 2NY (GB)**
- **SWANSON, Stephen, GlaxoSmithKline Stevenage, Hertfordshire SG1 2NY (GB)**
- **WALKER, Andrew, John, GlaxoSmithKline Stevenage, Hertfordshire SG1 2NY (GB)**
- **WILKINSON, Mark, GlaxoSmithKline Stevenage, Hertfordshire SG1 2NY (GB)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(74) Representative: **Povey, Alexander W.G.**
**GlaxoSmithKline**
**Corporate Intellectual Property**
**(CN9.25.1)**
**980 Great West Road**
**Brentford, Middlesex TW8 9GS (GB)**

(56) References cited:
**EP-A- 0 243 959**
**WO-A-00/35877**
**WO-A-02/26722**
**WO-A-02/26723**
**US-A- 5 405 854**

## Description

This invention relates to a novel compound, a process for its preparation, pharmaceutical formulations containing it and its use in therapy.

Inflammation is a primary response to tissue injury or microbial invasion and is characterised by leukocyte adhesion to the endothelium, diapedesis and activation within the tissue. Leukocyte activation can result in the generation of toxic oxygen species (such as superoxide anion), and the release of granule products (such as peroxidases and proteases). Circulating leukocytes include neutrophils, eosinophils, basophils, monocytes and lymphocytes. Different forms of inflammation involve different types of infiltrating leukocytes, the particular profile being regulated by the profile of adhesion molecule, cytokine and chemotactic factor expression within the tissue.

The primary function of leukocytes is to defend the host from invading organisms, such as bacteria and parasites. Once a tissue is injured or infected, a series of events occurs which causes the local recruitment of leukocytes from the circulation into the affected tissue. Leukocyte recruitment is controlled to allow for the orderly destruction and phagocytosis of foreign or dead cells, followed by tissue repair and resolution of the inflammatory infiltrate. However in chronic inflammatory states, recruitment is often inappropriate, resolution is not adequately controlled and the inflammatory reaction causes tissue destruction.

There is increasing evidence that the bronchial inflammation which is characteristic of asthma represents a specialised form of cell-mediated immunity, in which cytokine products, such as IL-4 and IL-5 released by T-helper 2 (Th2) lymphocytes, orchestrate the accumulation and activation of granulocytes, in particular eosinophils and to a lesser extent basophils. Through the release of cytotoxic basic proteins, pro-inflammatory mediators and oxygen radicals, eosinophils generate mucosal damage and initiate mechanisms that underlie bronchial hyperreactivity. Therefore, blocking the recruitment and activation of Th2 cells and eosinophils is likely to have anti-inflammatory properties in asthma. In addition, eosinophils have been implicated in other disease types such as rhinitis, eczema, irritable bowel syndrome and parasitic infections.

Chemokines are a large family of small proteins which are involved in trafficking and recruitment of leukocytes (for review see Luster, New Eng. J. Med., 338, 436-445 (1998)). They are released by a wide variety of cells and act to attract and activate various cell types, including eosinophils, basophils, neutrophils, macrophages, T and B lymphocytes. There are two major families of chemokines, CXC- ($\alpha$) and CC- ($\beta$) chemokines, classified according to the spacing of two conserved cysteine residues near to the amino terminus of the chemokine proteins. Chemokines bind to specific cell surface receptors belonging to the family of G-protein-coupled seven transmembrane-domain proteins (for review see Luster, 1998). Activation of chemokine receptors results in, amongst other responses, an increase in intracellular calcium, changes in cell shape, increased expression of cellular adhesion molecules, degranulation and promotion of cell migration (chemotaxis).

To date a number of CC chemokine receptors have been identified and of particular importance to the current invention is the CC-chemokine receptor-3 (CCR-3), which is predominantly expressed on eosinophils, and also on basophils, mast cells and Th2 cells. Chemokines that act at CCR-3, such as RANTES, MCP-3 and MCP-4, are known to recruit and activate eosinophils. Of particular interest are eotaxin and eotaxin-2, which specifically bind to CCR-3. The localization and function of CCR-3 chemokines indicate that they play a central role in the development of allergic diseases such as asthma. Thus, CCR-3 is specifically expressed on all the major cell types involved in inflammatory allergic responses. Chemokines that act at CCR-3 are generated in response to inflammatory stimuli and act to recruit these cell types to sites of inflammation, where they cause their activation (e.g. Griffiths et al., J. Exp. Med., 179, 881-887 (1994), Lloyd et al., J. Exp. Med., 191, 265-273 (2000)). In addition, anti-CCR-3 monoclonal antibodies completely inhibit eotaxin interaction with eosinophils (Heath, H. et al., J. Clin. Invest. 99 (2), 178-184 (1997)), while an antibody for the CCR-3 specific chemokine, eotaxin, reduced both bronchial hyperreactivity and lung eosinophilia in an animal model of asthma (Gonzalo et al., J. Exp. Med., 188, 157-167 (1998). Thus, many lines of evidence indicate that antagonists at the CCR-3 receptor are very likely to be of therapeutic use for the treatment of a range of inflammatory conditions.

In addition to a key role in inflammatory disorders, chemokines and their receptors also play a role in infectious disease. Mammalian cytomegaloviruses, herpes viruses and pox viruses express chemokine receptor homologues, which can be activated by human CC chemokines such as RANTES and MCP-3 receptors (for review see Wells and Schwartz, Curr. Opin. Biotech., 8, 741-748, 1997). In addition, human chemokine receptors, such as CXCR-4, CCR-5 and CCR-3, can act as co-receptors for the infection of mammalian cells by microbes such as human immunodeficiency viruses (HIV). Thus, chemokine receptor antagonists, including CCR-3 antagonists, may be useful in blocking infection of CCR-3 expressing cells by HIV or in preventing the manipulation of immune cellular responses by viruses such as cytomegaloviruses.

International Patent Application publication number WO 01/24786 (Shionogi & Co. Ltd.) discloses certain aryl and heteroaryl derivatives for treating diabetes. WO 00/69830 (Torrey Pines Institute for Molecular Studies) discloses certain diazacyclic compounds, and libraries containing them, for biological screening. WO 00/18767 (Neurogen Corporation) discloses certain piperazine derivatives as dopamine D4 receptor antagonists. United States Patent 6,031,097

and WO 99/21848 (Neurogen Corporation) discloses certain aminoisoquinoline derivatives as dopamine receptor ligands. WO 99/06384 (Recordati Industria Chimica) discloses piperazine derivatives useful for the treatment of neuromuscular dysfunction of the lower urinary tract. WO 98/56771 (Schering Aktiengesellschaft) discloses certain piperazine derivatives as anti-inflammatory agents. WO 97/47601 (Yoshitomi Pharmaceutical Industries Ltd.) discloses certain fused heterocyclic compounds as dopamine D-receptor blocking agents. WO 96/39386 (Schering Corporation) discloses certain piperidine derivatives as neurokinin antagonists. WO 96/02534 (Byk Gulden Lomberg Chemische Fabrik GmbH) discloses certain piperazine thiopyridines useful for controlling helicobacter bacteria. WO 95/32196 (Merck Sharp & Dohme Limited) discloses certain piperazine, piperidine, and tetrahydropyridine derivatives as 5-HT1 D-alpha antagonists. United States Patent 5,389,635 (E.I. Du Pont de Nemours and Company) discloses certain substituted imadazoles as angiotensin-II antagonists. European Patent Application publication number 0 306 440 (Schering Aktiengesellschaft) discloses certain imidazole derivatives as cardiovascular agents.

A novel compound has now been found which is a CCR-3 antagonist. This compound blocks the migration/chemotaxis of eosinophils and thus possesses anti-inflammatory properties. This compound is therefore of potential therapeutic benefit, especially in providing protection from eosinophil, basophil and Th2-cell-induced tissue damage in diseases where such cell types are implicated, particularly allergic diseases, including but not limited to bronchial asthma, allergic rhinitis and atopic dermatitis.

Thus, according to one aspect of the invention, there is provided a compound of formula (I):

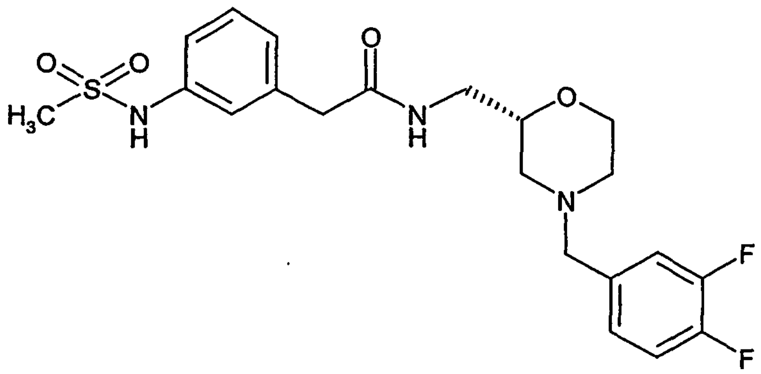

(I)

which is;

N-{[(2S)-4-(3,4-difluorobenzyl)morpholin-2-yl]methyl}-2-{3-[(methylsulfonyl)amino]phenyl}acetamide;

and salts and solvates thereof.

Suitable salts of the compound of formula (I) include physiologically acceptable salts and salts which may not be physiologically acceptable but may be useful in the preparation of compounds of formula (I) and physiologically acceptable salts thereof. If appropriate, acid addition salts may be derived from inorganic or organic acids, for example hydrochlorides, hydrobromides, sulphates, phosphates, acetates, benzoates, citrates, succinates, lactates, tartrates, fumarates, maleates, 1-hydroxy-2-naphthoates, pamoates, methanesulphonates, formates or trifluoroacetates.

Examples of solvates include hydrates.

The compound of formula (I) and salts and solvates thereof may be prepared by the methodology described hereinafter, constituting a further aspect of this invention.

A process according to the invention for the preparation of the compound of formula (I) comprises coupling of a compound of formula (II) or a salt thereof:

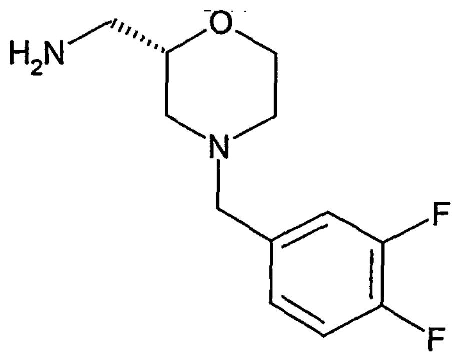

(II)

with a compound of formula (III):

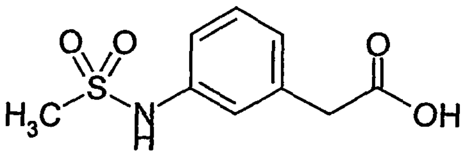

(III)

and thereafter, if required, carrying out one or more of the following optional steps:

(i) removing any protecting group(s);
(ii) preparing an appropriate salt or solvate of the compound so formed.

The coupling of the compounds of formulae (II) and (III) is carried out in any suitable solvent, for example a polar organic solvent such as N,N-dimethylformamide in the presence of a suitable dehydrating agent such as a carbodiimide reagent e.g. 1-(3-dimethylaminopropyl)-3-ethylcarbodiimide hydrochloride, and optionally a suitable activating agent, e.g. an activated hydroxy compound such as 1-hydroxybenzotriazole, and optionally in the presence of a base such as a tertiary amine, e.g. N,N-diisopropylethylamine, under conventional coupling conditions at any temperature providing a suitable rate of formation of the required product, over a suitable reaction time, for example 12 - 24 hours.

Suitable reaction temperatures include those in the range of 18°C to 25°C.

The reaction products are isolated and purified using conventional methods.

The compound of formula (III) may be prepared by sulphonylation of the compound of formula (IV):

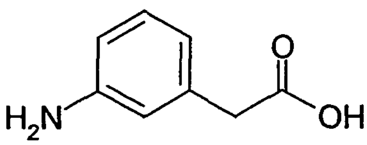

(IV)

The sulphonylation may be carried out using a conventional sulphonylating agent, for example a sulphonyl chloride such as methanesulphonyl chloride, in the presence of a suitable base, for example aqueous sodium carbonate.

The compound of formula (II) may be prepared by either by Reaction (a), Reaction (b), or Reaction (c).

Reaction (a). Reaction of the compound of formula (V) with a compound of formula (VI)

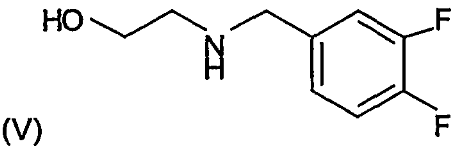

(V)

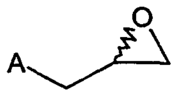

(VI)

wherein A is a protected amino group, suitably phthalimido, followed by deprotection of the amino group to give a compound of formula (IIR)

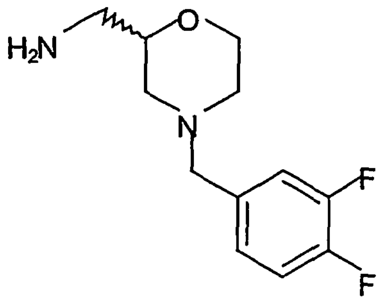

(IIR)

followed by resolution of the resulting enantiomers of the compound of formula (IIR);
or;
Reaction (b). Reaction of a compound of formula (V) as hereinbefore defined with a compound of formula (VIA)

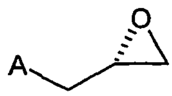

(VIA)

wherein A is as hereinbefore defined for formula (VI), followed by deprotection of the amino group to give the compound of formula (II).
Reaction (c). Hydrolysis of the compound of formula (VII);

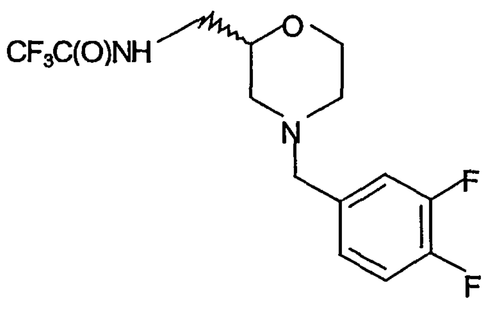

(VII)

followed by resolution of the resulting enantiomers of a compound of formula (IIR).

For both reactions (a) and (b), the cyclisation of the intermediate diols (IIBR) and (IIB) in the reaction between the compound of formula (V) and a compound of formula (VI) or (VIA) is typically carried out under the Mitsunobu conditions as follows:

Typically, a mixture of the compound of formula (V) and the compound of formula (VI) or formula (VIA) in a suitable solvent, such as tetrahydrofuran, is stirred, suitably for 20-24 hours at a suitable temperature, suitably the reflux temperature of the solvent, under an inert atmosphere, suitably an atmosphere of nitrogen. Further solvent is then added

and the mixture cooled, suitably to 0-5°C. A suitable phosphine, suitably triphenyl phosphine, is added and the mixture stirred until all the solid is dissolved. A suitable azo compound, suitably diisopropylazodicarboxylate, is then added over a period of time, suitably, 10-15 minutes, while maintaining the temperature at <7°C. The mixture is allowed to stand for a period of time, suitably 2-3 hours, then allowed to warm, suitably to 20-25°C. After a further period of standing, suitably 4-6 hours, further phosphine and azo compound are added. After a further period of standing, suitably 20-24 hours, the reaction mixture is concentrated to near dryness. A suitable alcohol, suitably propan-2-ol, is added and the concentration step repeated; the alcohol addition and concentration step is then repeated. Further alcohol is then added and the mixture heated to a temperature suitably between 65-75C°. After a suitable period, suitably 20-45 minutes, the resultant slurry is cooled suitably to 20-25°C, and then allowed to stand, suitably for 1.5 - 3 hours, after which time the product is isolated by filtration. The filter bed is washed with more alcohol and then dried *in vacuo* at 35-45°C to yield the protected form of the compound of formula (IIR) or formula (II) respectively.

The removal of the protecting group from the product is typically carried out as follows. A slurry of the protected form of the compound of formula (IIR) or formula (II) in an appropriate polar solvent, suitably water, is heated to elevated temperature, suitably 70-75°C and then treated dropwise with a concentrated mineral acid, suitably concentrated sulphuric acid. The mixture was then heated at elevated temperature, suitably the reflux temperature of the solvent, for a suitable period of time, suitably 20-24 hours, after which the reaction mixture was cooled to 20-25°C and then treated with a suitable apolar solvent, suitably dichloromethane. A base, suitably 0.880 ammonia solution, is then added dropwise, maintaining the temperature between 20-25°C. Further apolar solvent is then added, the aqueous phase then being separated and extracted with further apolar solvent. The combined organic phase is washed with water and then evaporated to dryness. The residue is redissolved and the apolar solvent evaporated to give the compound of formula (IIR) or formula (II).

The process for the preparation of the protected form of the compound of formula (IIR) or formula (II) described above may also be undertaken in two stages, in which an intermediate compound of formula (IIBR) or of formula (IIB) respectively;

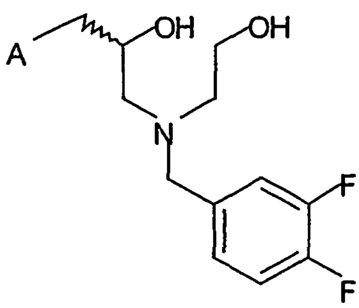 (IIBR) 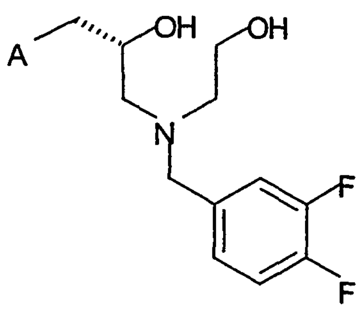 (IIB)

wherein A is as hereinbefore defined for formulae (VI) and (VIA); is isolated.

Typically, a mixture of the compound of formula (V) and a compound of formula (VI) or formula (VIA) in a suitable solvent, such as tetrahydrofuran, is stirred, suitably for 20-24 hours at a suitable temperature, suitably the reflux temperature of the solvent, under an inert atmosphere, suitably an atmosphere of nitrogen. Further compound of formula (V) is added and the mixture heated at a suitable temperature, suitably the reflux temperature of the solvent, under an inert atmosphere, suitably an atmosphere of nitrogen, for a suitable period of time, suitably 3-6 hours. The reaction mixture is then cooled, suitably to 20-25°C, and the compound precipitated by means of addition of a suitable co-solvent, suitably diisopropyl ether. The compound of formula (IIBR) or formula (IIB) respectively is isolated by filtration, washed with further co-solvent and dried *in vacuo.*

A protected form of the compound of formula (IIR) or formula (II) may then be prepared from a compound of formula (IIBR) or formula (IIB) under similar conditions to those of the reaction between a compound of formula (V) and formulae (VI) or (VIA) as hereinbefore described, but omitting the reflux period prior to the addition of the phosphine and azo compounds.

Reaction (c) is typically carried out by stirring a solution of the compound of formula (VII) in a suitable solvent, for example a mixture of methanol and water, and adding a suitable base, for example potassium carbonate. The mixture is stirred at a suitable temperature, for example those in the range 20-25°C for a suitable time, for example 16-20 hours followed by removal of the organic solvent in vacuo. Water is then added and the mixture extracted with a suitable organic solvent, for example ethyl acetate. The combined organic phases are washed with water and saturated aqueous sodium chloride solution before drying over a suitable drying agent, for example sodium sulphate, filtering and evaporation of the solvent in vacuo. The crude product is then purified by flash chromatography.

The resolution of the compound of formula (II) from the racemic product i.e. the compound of formula (IIR) may be undertaken using techniques well known to those skilled in the art, for example preparative chiral high performance liquid chromatography (chiral HPLC) or by fractional crystallisation of diastereoisomeric salts.

The compound of formula (V) may be prepared from the compound of formula (IX)

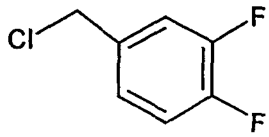

(IX)

by reaction with ethanolamine. Suitably the reaction is carried out at elevated temperature, e.g. 40 - 60°C, in the absence of solvent.

The compound of formula (VII) may be prepared by reaction of the compound of formula (VIII)

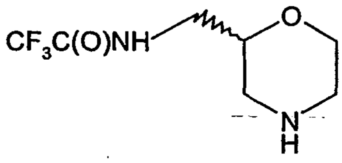

(VIII)

with 3,4-difluorobenzyl chloride.

The reaction between the compound of formula (VIII) and 3,4-difluorobenzyl chloride is typically carried out in a suitable solvent, for example N,N-dimethylformamide, under an inert atmosphere, for example an atmosphere of nitrogen, with the addition of a suitable base, for example potassium carbonate, and a suitable activating agent, such as sodium iodide. The mixture is then stirred at a suitable temperature, for example a temperature in the range of 20-25°C, for a suitable period of time, for example 16-20 hours before removing the volatile components in vacuo.

The compound of formula (VIII) is prepared by treating a solution of morpholin-2-ylmethylamine in a suitable organic solvent, for example methanol, under an inert atmosphere, for example an atmosphere nitrogen, with a solution of ethyl-α,α,α-trifluoroacetate in a suitable organic solvent, for example ether. The mixture is then stirred for a suitable period of time, for example 20-40 minutes at a suitable temperature, for example a temperature in the range of 20-25°C and the volatile components removed in vacuo. The residue is then dissolved in a suitable organic solvent, for example methanol, and the volatile components removed in vacuo.

The compounds of formula (IV) and (VI) are commercially available compounds and may be prepared by analogy with known procedures, for example those disclosed in in standard reference texts of synthetic methodology such as J. March, Advanced Organic Chemistry, 3rd Edition (1985), Wiley Interscience.

The compounds of formulae (II), (IIBR), (IIB) and (V) are considered to be novel.

Accordingly, there is provided a compound of formula (II).

There is also provided a compound of formula (IIBR).

There is also provided a compound of formula (IIB).

There is also provided a compound of formula (V).

Suitable protecting groups in any of the above mentioned reactions are those used conventionally in the art. The methods of formation and removal of such protecting groups are those conventional methods appropriate to the molecule being protected, for example those methods discussed in standard reference texts of synthetic methodology such as P J Kocienski, Protecting Groups, (1994), Thieme.

For any of the hereinbefore described reactions or processes, conventional methods of heating and cooling may be employed, for example electric hotplates and ice/salt baths respectively. Conventional methods of purification, for example crystallisation and column chromatography may be used as required.

The salts and solvates of the compounds of formula (I) may be prepared from the compound of formula (I) or suitable salts or solvates thereof and isolated according to conventional procedures.

The compounds of the invention may be tested for *in vitro* biological activity in accordance with the following assays:

(a) CCR-3 Binding Assay

A CCR-3 competition binding SPA (scintillation proximity assay) was used to assess the affinity of novel compounds for CCR-3. Membranes prepared from K562 cells stably expressing CCR-3 (2.5μg/well) were mixed with 0.25mg/well wheat-germ agglutinin SPA beads (Amersham) and incubated in binding buffer (HEPES 50 mM, $CaCl_2$ 1 mM, $MgCl_2$ 5 mM, 0.5% BSA) at 4°C for 1.5 hr. Following incubation, 20 pM of [$^{125}$I] eotaxin (Amersham) and increasing concentrations of compound (1 pM to 30μM) were added and incubated in a 96 well plate for 2 hr at 22°C then counted on a Microbeta plate counter. The total assay volume was 100 μl. Competition binding data were analysed by fitting the data with a four parameter logistic equation. Data are presented as the mean $pIC_{50}$ values (negative logarithm of the concentration of compound which inhibits [$^{125}$I]eotaxin binding by 50%) from at least two experiments.

(b) Eosinophil Chemotaxis Assay.

The compound was evaluated for its inhibitory effect on eosinophil chemotaxis. Eosinophils were purified from human peripheral blood by standard CD16 cell depletion using a Miltenyi cell separation column and a magnetic Super Macs magnet as previously described (Motegi & Kita, 1998; J.Immunology. 161:4340-6). Cells were re-suspended in RPMI 1640/10% FCS solution and incubated with calcein-AM (Molecular Probes) at 37°C for 30 mins. Following incubation, the eosinophils were centrifuged at 400g for 5 min and re-suspended in RPMI/FCS at 2.2 million/ml. Cells were then incubated in the presence of increasing concentration of compounds (1 pM to 30 μM) at 37°C for 30 mins. For control responses cells were incubated with RPMI/FCS only. The agonist eotaxin (an $EC_{80}$ concentration) was added to the lower chamber of a 96 well chemotaxis plate (5 μm filter: Receptor Technologies). Eosinophils (50 μl of 2 million/ml cells) were added to the top chamber of the filter plate and incubated at 37°C for 45 mins. Cells remaining on top of the chemotaxis filter were removed and the number of eosinophils which had migrated were quantified by reading the plate on a fluorescent plate reader. Inhibition curves for the effect of compounds on eosinophil chemotaxis were analysed by fitting the data with a four parameter logistic equation. Functional $pK_i$ values ($fpK_i$) were generated using the equation below (Lazareno & Birdsall, 1995. Br.J.Pharmacol 109: 1110-9).

$$fpKi = \frac{IC_{50}}{1 + \frac{[Agonist]}{EC_{50}}}$$

The compound of the invention was tested in the CCR-3 binding and/or eosinophil chemotaxis assays (assays (a) and (b)). The compound of the invention was tested in the CCR-3 binding assay and possessed a pIC50 value of 8.0. The compound of the invention tested in the CCR-3 eosinophil chemotaxis assay possessed an fpKi value of 8.4.

Examples of disease states in which the compound of the invention has potentially beneficial anti-inflammatory effects include diseases of the respiratory tract such as bronchitis (including chronic bronchitis), bronchiectasis, asthma (including allergen-induced asthmatic reactions), chronic obstructive pulmonary disease (COPD), cystic fibrosis, sinusitis and rhinitis.

Also included are diseases of the gastrointestinal tract such as intestinal inflammatory diseases including inflammatory bowel disease (e.g. Crohn's disease or ulcerative colitis) and intestinal inflammatory diseases secondary to radiation exposure or allergen exposure.

Furthermore, the compound of the invention may be used to treat nephritis; skin diseases such as psoriasis, eczema, allergic dermatitis and hypersensitivity reactions; and diseases of the central nervous system which have an inflammatory component (eg. Alzheimer's disease, meningitis, multiple sclerosis), HIV and AIDS dementia.

The compounds of the present invention may also be of use in the treatment of nasal polyposis, conjunctivitis or pruritis.

Further examples of disease states in which the compound of the invention has potentially beneficial effects include cardiovascular conditions such as atherosclerosis, peripheral vascular disease and idiopathic hypereosinophilic syndrome.

The compounds of the invention may be useful as an immunosuppressive agents and so has use in the treatment of auto-immune diseases such as allograft tissue rejection after transplantation, rheumatoid arthritis and diabetes. The compound of the invention may also be useful in inhibiting metastasis. Diseases of principal interest include asthma, COPD and inflammatory diseases of the upper respiratory tract involving seasonal and perennial rhinitis.

It will be appreciated by those skilled in the art that reference herein to treatment extends to prophylaxis as well as the treatment of established conditions.

As mentioned above, the compound of formula (I) is useful as a therapeutic agent.

There is thus provided as a further aspect of the invention a compound of formula (I) or a physiologically acceptable salt or solvate thereof for use as an active therapeutic agent.

There is also therefore provided the compound of formula (I), or a physiologically acceptable salt or solvate thereof, for use in the treatment of inflammatory conditions, eg. asthma or rhinitis.

According to another aspect of the invention, there is provided the use of the compound of formula (I) or a physiologically acceptable salt or solvate thereof for the manufacture of a medicament for the treatment of patients with inflammatory conditions, eg. asthma or rhinitis.

Disclosed is a method for the treatment of a human or animal subject with an inflammatory condition eg. asthma or rhinitis, which method comprises administering an effective amount of the compound of formula (I) or a physiologically acceptable salt or solvate thereof.

The compound according to the invention may be formulated for administration in any convenient way.
There is thus further provided a pharmaceutical composition comprising the compound of formula (I), or a physiologically acceptable salt or solvate thereof, and optionally one or more physiologically acceptable diluents or carriers.

There is also provided a process for preparing such a pharmaceutical formulation which comprises admixing the compound of formula (I) or a physiologically acceptable salt or solvate thereof with one or more physiologically acceptable diluents or carriers.

The compound according to the invention may, for example, be formulated for oral, inhaled, intranasal, buccal, parenteral or rectal administration, preferably for oral administration.

Tablets and capsules for oral administration may contain conventional excipients such as binding agents, for example syrup, acacia, gelatin, sorbitol, tragacanth, mucilage of starch, cellulose or polyvinyl pyrrolidone; fillers, for example, lactose, microcrystalline cellulose, sugar, maize- starch, calcium phosphate or sorbitol; lubricants, for example, magnesium stearate, stearic acid, talc, polyethylene glycol or silica; disintegrants, for example, potato starch, croscarmellose sodium or sodium starch glycollate; or wetting agents such as sodium lauryl sulphate. The tablets may be coated according to methods well known in the art.

Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, for example, sorbitol syrup, methyl cellulose, glucose/sugar syrup, gelatin, hydroxymethyl cellulose, carboxymethyl cellulose, aluminium stearate gel or hydrogenated edible fats; emulsifying agents, for example, lecithin, sorbitan mono-oleate or acacia; non-aqueous vehicles (which may include edible oils), for example almond oil, fractionated coconut oil, oily esters, propylene glycol or ethyl alcohol; or preservatives, for example, methyl or propyl p- hydroxybenzoates or sorbic acid. The preparations may also contain buffer salts, flavouring, colouring and/or sweetening agents (e.g. mannitol) as appropriate.

For buccal administration the compositions may take the form of tablets or lozenges formulated in conventional manner.

The compound may also be formulated as suppositories, e.g. containing conventional suppository bases such as cocoa butter or other glycerides.

The compound according to the invention may also be formulated for parenteral administration by bolus injection or continuous infusion and may be presented in unit dose form, for instance as ampoules, vials, small volume infusions or pre-filled syringes, or in multidose containers with an added preservative. The compositions may take such forms as solutions, suspensions, or emulsions in aqueous or non-aqueous vehicles, and may contain formulatory agents such as anti-oxidants, buffers, antimicrobial agents and/or tonicity adjusting agents. Alternatively, the active ingredient may be in powder form for constitution with a suitable vehicle, e.g. sterile, pyrogen-free water, before use. The dry solid presentation may be prepared by filling a sterile powder aseptically into individual sterile containers or by filling a sterile solution aseptically into each container and freeze-drying.

The compound and pharmaceutical compositions according to the invention may also be used in combination with other therapeutic agents, for example antihistaminic agents, anticholinergic agents, anti-inflammatory agents such as corticosteroids, e.g. fluticasone propionate, beclomethasone dipropionate, mometasone furoate, triamcinolone acetonide or budesonide; or non-steroidal anti-inflammatory drugs (NSAIDs) eg. sodium cromoglycate, nedocromil sodium, PDE-4 inhibitors, leukotriene antagonists, iNOS inhibitors, tryptase and elastase inhibitors, beta-2 integrin antagonists and adenosine 2a agonists; or beta adrenergic agents such as salmeterol, salbutamol, formoterol, fenoterol or terbutaline and salts thereof; or antiinfective agents e.g. antibiotic agents and antiviral agents. It will be appreciated that when the compound of the present invention is administered in combination with other therapeutic agents normally administered by the inhaled or intranasal route, that the resultant pharmaceutical composition may be administered by the inhaled or intranasal route.

The compounds of the invention may conveniently be administered in amounts of, for example, 0.001 to 500mg/kg body weight, preferably 0.01 to 500mg/kg body weight, more preferably 0.01 to 100mg/kg body weight, and at any appropriate frequency e.g. 1 to 4 times daily. The precise dosing regimen will of course depend on factors such as the therapeutic indication, the age and condition of the patient, and the particular route of administration chosen.

Throughout the description and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

General Experimental Details

Liquid Chromatography Mass Spectrometry (LC/MS) System

The following (LC/MS) system was used:

an 3μm ABZ+PLUS (3.3cm x 4.6mm internal diameter) column, eluting with solvents:A - 0.1%v/v formic acid + 0.077% w/v ammonium acetate in water; and
B - 95:5 acetonitrile:water + 0.05%v/v formic acid, at a flow rate of 3 ml per minute. The following gradient protocol was used: 100% A for 0.7mins; A+B mixtures, gradient profile 0 - 100% B over 3.5mins; hold at 100%B for 1.1mins; return to 100% A over 0.2mins.

Solid phase extraction (ion exchange)

'SCX' refers to Isolute Flash SCX-2 sulphonic acid solid phase extraction cartridges.
All temperatures are in °C

Example 1: N-{[(2S)-4-(3,4-difluorobenzyl)morpholin-2-yl]methyl}-2-{3-[(methylsulfonyl)amino]phenyl}acetamide

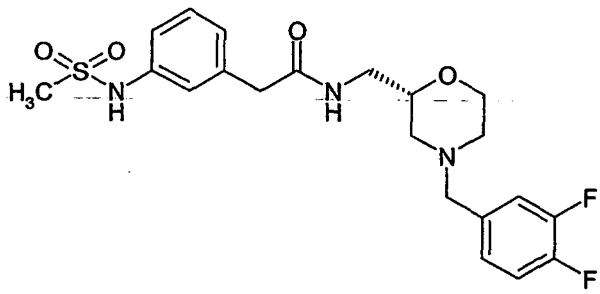

To a stirred solution of Description 4 (0.0242g) in N,N-dimethylformamide (1ml) was added a solution of 1-(3-dimethylaminopropyl)-3-ethylcarbodiimide hydrochloride (0.0304g), 1-hydroxybenzotriazole (0.0171g), and Description 3 (0.0243g) in N,N-dimethylformamide (1ml) at 22°. N,N-diisopropylethylamine (0.0368ml) was added to the mixture which was then stirred at 22° for 18h. The mixture was applied to a 2g SCX ion-exchange cartridge (IST Isolute Flash SCX-2), pre-conditioned with methanol. The cartridge was eluted with methanol and 10% 0.880 ammonia solution in methanol. The first ammonia fraction was evaporated *in vacuo* and the residue was further purified by Biotage™ flash chromatography on silica gel, eluting with 200:8:1 dichloromethane/ethanol/0.880 ammonia solution. The required fractions were combined and the solvent evaporated *in vacuo* to give the title compound (0.0353g) as a colourless glass. LC/MS : $R_t$ = 2.16min, *m/z* 454 [$MH^+$]

Description 1: 2-(3,4-Difluorobenzylamino)-ethanol

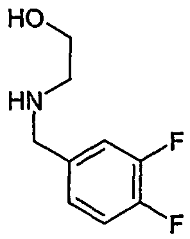

4-Chloromethyl-1,2-difluorobenzene (90.0g) (Fluorochem chemicals/Journal of Organic Chemistry, 1961, (26), 2353-2355) was added dropwise to ethanolamine (325.8ml) at 40°C, with stirring under nitrogen and cooling to maintain the reaction temperature. The mixture was stirred for 3h whilst being heated at approximately 50°C before being cooled to room temperature. The mixture was partitioned between ethyl acetate and saturated aqueous sodium bicarbonate solution. The phases were separated and the organic phase was washed with further saturated aqueous sodium bicarbonate solution (100ml). The combined aqueous phases were further extracted with a portion of ethyl acetate (100ml) and the combined organic phases were washed with water (2x500ml) and saturated aqueous sodium chloride solution. The organic phase was concentrated *in vacuo* and re-concentrated after addition of toluene to give the title compound as a white solid (92.5g).
Mass Spectum *m*/*z* 188 [$MH^+$]

Description 2: [4-(3,4-Difluorobenzyl)morpholin-2-yl]methylamine)

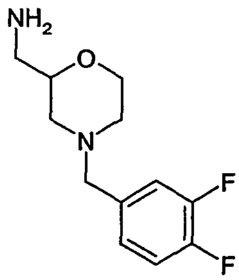

2-(Oxiran-2-ylmethyl)-1H-isoindole-1,3(2H)-dione (166.8g) was added portionwise to the stirred compound of Description 1 (128.12g) at 80-90°C under nitrogen over 50mins. The mixture was stirred at 80-90°C for a further 3h before concentrated sulphuric acid (200ml) was added dropwise over 40mins. The mixture was heated to 150°C and stirred overnight. After cooling to room temperature, the mixture was washed with ethyl acetate (2x400ml). The aqueous phase was cooled to 10°C and saturated aqueous sodium chloride (400ml) was added carefully over 30mins. Further saturated aqueous sodium chloride (400ml) was added to the aqueous phase and the mixture was washed with further portions of ethyl acetate (3x500ml) The aqueous phase was cooled to 10°C and adjusted to pH 12-13 with 10N aqueous sodium hydroxide. The mixture was extracted with ethyl acetate (3x500ml) and the combined organic phases were filtered through 'Hyflo' filter aid. The combined organic extracts were washed with water (3x500ml) and saturated aqueous sodium chloride. The organic phase was concentrated *in vacuo* and re-concentrated (x3) after addition of toluene (3x150ml) to give the title compound as an amber oil (94g).
Mass Spectum *m*/*z* 243 [$MH^+$]

Description 3: 1-[(2S)-4-(3,4-Difluorobenzyl)morpholin-2-yl]methylamine

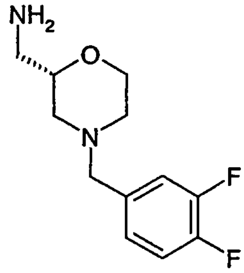

Description 2 (4.5g) (racemic mixture) was separated into its single enantiomers by preparative chiral-HPLC. The separation was carried out using a 2" x 22cm Chiralpak AD 20μm column, Merck self pack DAC system, eluting with 95:5:0.1 (v/v) heptane : absolute ethanol : diethylamine (flow rate: 50ml/min over 40min, UV detection 220nm); sample load preparation: 300mg sample in 10ml 1:1 (v/v) absolute ethanol : system eluent. The solvent was removed from the required fractions *in vacuo* and the product was partitioned between dichloromethane and 2N aqueous hydrochloric acid, the phases separated and the organic phase extracted with further 2N aqueous hydrochloric acid (x2). The combined aqueous extracts were adjusted to pH12 by the addition of solid potassium carbonate and the solution was saturated with solid sodium chloride. The solution was extracted with dichloromethane (x3) and the combined organic extracts washed with water, dried over magnesium sulphate, filtered and the solvent removed *in vacuo* to give the title compound (1.84g) as a yellow oil. Preparative HPLC retention time 28.5mins

Description 4: 3-[(methylsulphonyl)amino]phenylacetic acid

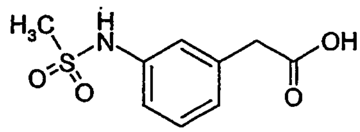

To a stirred solution of 3-aminophenylacetic acid (3.2g) and sodium carbonate (5.44g) in de-ionised water (36ml) was added methanesulphonyl chloride (1.7 ml). The mixture was heated with stirring at 85° for 4h before being left to cool to room temperature, acidified to pH 2 with conc. hydrochloric acid and left to stand in a refrigerator at 4° overnight. The precipitated solid was filtered, washed with water and ether and the combined filtrate and washings were evaporated to dryness *in vacuo*. The resultant residue was dissolved in hot water and left to recrystallise overnight whilst standing in a refrigerator at 4°C. The crystals were filtered, washed with a small quantity of cold water and dried *in vacuo* to give the title compound (0.417g) as colourless crystals.
LC/MS : $R_t$ = 2.00min, *m/z* 228 [$MH^-$], *m/z* 247 [$MNH_4^+$]

## Claims

**1.** A compound of formula (I):

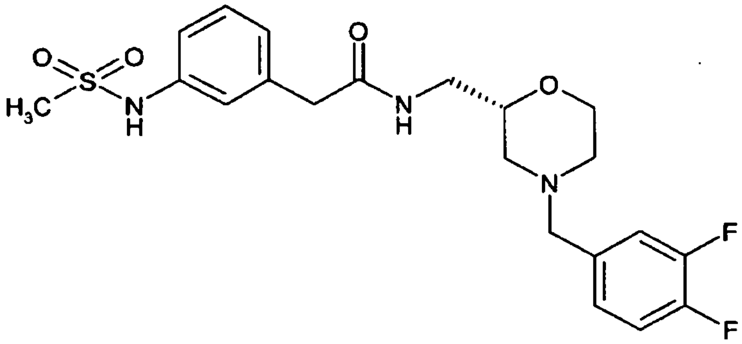

(I)

and salts and solvates thereof.

2. A process for the preparation of a compound of formula (I) or a salt or solvate thereof which process comprises coupling of a compound of formula (II) or a salt thereof with a compound of formula (III)

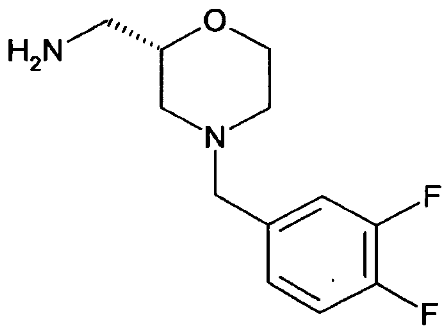

(II)

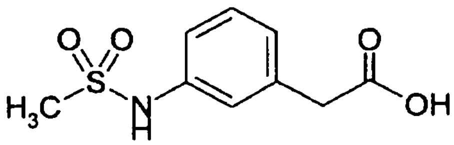

(III)

3. A compound as claimed in claim 1 for use as a therapeutic agent

4. The use of a compound as claimed in claim 1 in the manufacture of a medicament for the treatment of a human or animal subject suffering from, or susceptible, to an inflammatory condition.

5. A pharmaceutical composition comprising a compound as claimed in claim 1 and a physiologically acceptable carrier therefor.

6. A compound of formula (IIBR)

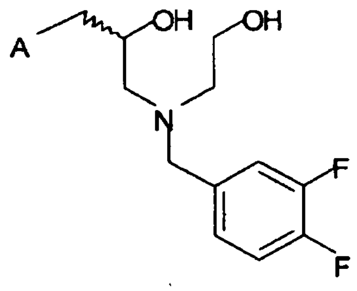

wherein A is a protected amino group.

**7.** A compound of formula (IIB)

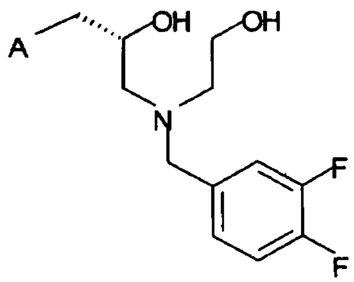

(IIB)

wherein A is a protected amino group.

**8.** A compound of formula (V)

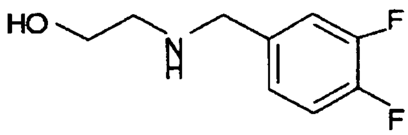

(V)

**Patentansprüche**

**1.** Verbindung der Formel (I):

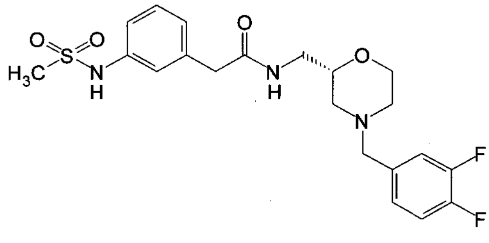

(I)

und Salze und Solvate davon.

**2.** Verfahren zur Herstellung einer Verbindung der Formel (I) oder eines Salzes oder Solvats davon, wobei das Verfahren das Kuppeln einer Verbindung der Formel (II) oder eines Salzes davon mit einer Verbindung der Formel (III)

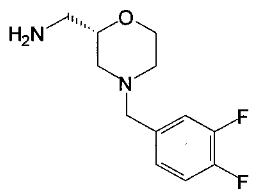

( II )

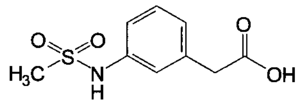

(I II )

umfasst.

3. Verbindung wie in Anspruch 1 beansprucht, zur Verwendung als therapeutisches Mittel.

4. Verbindung einer Verbindung, wie in Anspruch 1 beansprucht, für die Herstellung eines Medikaments zur Behandlung eines menschlichen oder tierischen Subjekts, das an entzündlichen Zuständen leidet oder für diese empfänglich ist.

5. Pharmazeutische Zusammensetzung, umfassend eine Verbindung, wie in Anspruch 1 beansprucht, und einen physiologisch annehmbaren Träger hierfür.

6. Verbindung der Formel (IIBR):

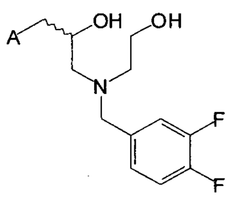

worin A eine geschützte Aminogruppe ist.

7. Verbindung der Formel (IIB):

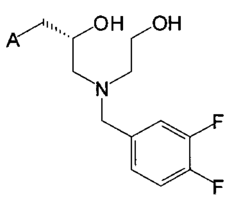

( IIB )

worin A eine geschützte Aminogruppe ist.

8. Verbindung der Formel (V)

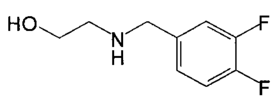

( V )

## Revendications

**1.** Composé de formule (I) :

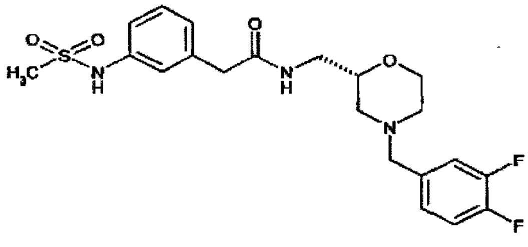

(I)

et des sels et des solvates de celui-ci.

**2.** Procédé de préparation d'un composé de formule (I) ou d'un sel ou d'un solvate de celui-ci, lequel procédé comprend le couplage d'un composé de formule (II) ou d'un sel de celui-ci avec un composé de formule (III)

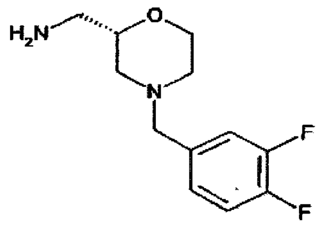

(II)

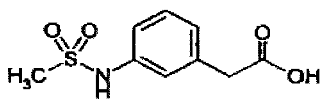

(III)

**3.** Composé selon la revendication 1, pour une utilisation en tant qu'agent thérapeutique.

**4.** Utilisation d'un composé selon la revendication 1 dans la fabrication d'un médicament destiné au traitement d'un sujet humain ou animal souffrant de, ou sensible à, une affection inflammatoire.

**5.** Composition pharmaceutique comprenant un composé selon la revendication 1 et un support physiologiquement acceptable pour celui-ci.

**6.** Composé de formule (IIBR)

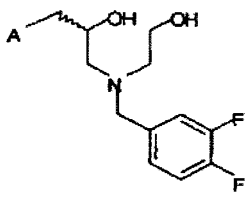

dans laquelle A représente un groupe amino protégé.

**7.** Composé de formule (IIB)

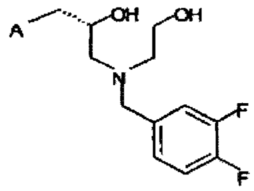

(IIB)

dans laquelle A représente un groupe amino protégé.

**8.** Composé de formule (V)

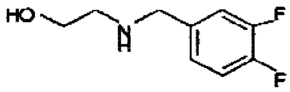

(V)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0124786 A
- WO 0069830 A
- WO 0018767 A
- US 6031097 A
- WO 9921848 A
- WO 9906384 A
- WO 9856771 A
- WO 9747601 A
- WO 9639386 A
- WO 9602534 A
- WO 9532196 A
- US 5389635 A
- EP 0306440 A


### Non-patent literature cited in the description


- **Luster.** *New Eng. J. Med.,* 1998, vol. 338, 436-445
- **Griffiths et al.** *J. Exp. Med.,* 1994, vol. 179, 881-887
- **Lloyd et al.** *J. Exp. Med.,* 2000, vol. 191, 265-273
- **Heath, H. et al.** *J. Clin. Invest.,* 1997, vol. 99 (2), 178-184
- **Gonzalo et al.** *J. Exp. Med.,* 1998, vol. 188, 157-167
- **Wells ; Schwartz.** *Curr. Opin. Biotech.,* 1997, vol. 8, 741-748
- **J. March.** Advanced Organic Chemistry. Wiley Interscience, 1985
- **P J Kocienski.** *Protecting Groups,* 1994
- **Motegi ; Kita.** *J.Immunology.,* 1998, vol. 161, 4340-6
- **Lazareno ; Birdsall.** *Br.J.Pharmacol,* 1995, vol. 109, 1110-9
- *Fluorochem chemicals/Journal of Organic Chemistry,* 1961, 2353-2355